Europäisches Patentamt

**European Patent Office**

Office européen des brevets

(11) Numéro de publication: **0 283 369 B1**

(12) **FASCICULE DE BREVET EUROPEEN**

(45) Date de publication de fascicule du brevet: **08.12.93** (51) Int. Cl.5: **A61K 31/155**

(21) Numéro de dépôt: **88400484.7**

(22) Date de dépôt: **02.03.88**

(54) **Emploi de la Metformine, dans la préparation de médicaments.**

(30) Priorité: **06.03.87 FR 8703028**

(43) Date de publication de la demande:
**21.09.88 Bulletin 88/38**

(45) Mention de la délivrance du brevet:
**08.12.93 Bulletin 93/49**

(84) Etats contractants désignés:
**AT BE CH DE FR GB IT LI LU NL SE**

(56) Documents cités:

Dialog Information Services, Base de Données 155: Medline 66-89, no. d'accès
01770275, G. Fearnley

Dialog Information Services, Base de Données 155: Medline 66-89, no. d'accès
04237731, S. Sgambato et al.

Dialog Information Services , Base de Données 155: Medline 66-89, no. d'accès
03566315, R. Cavaliere et al.

Dialog Information Services, Base de Données 155: Medline 66-89, no. d'accès
04284983, B. Baradli et al.

The LANCET; VOL: 1; NO: (/=; June 29, 1974,
p. 1341, C.W. Kenworthy

Thrombosis and Haemostasis, vol. 55, no. 3,
1986, pp. 361-5, O.G.K. Schattauer Verlag
GmbH, Stuttgart, DE, I. Peacock et al.

Gazette Médicale (1988) 95, no. 18, p. 68

(73) Titulaire: **LIPHA, LYONNAISE INDUSTRIELLE**
**PHARMACEUTIOUE**
**34, rue Saint Romain**
**Boîte Postale 8481**
**F-69359 Lyon Cedex 08(FR)**

(72) Inventeur: **Wiernsperger, Nicolas**
**Le Bonneton**
**Orlienas**
**F-69530 Brignais(FR)**
Inventeur: **Grand, Marcel**
**15, rue du Professeur Nicolas**
**F-69008 Lyon(FR)**

(74) Mandataire: **Moncheny, Michel et al**
**c/o Cabinet Lavoix**
**2 Place d'Estienne d'Orves**
**F-75441 Paris Cedex 09 (FR)**

**Description**

La présente invention concerne l'utilisation de la metformine et de ses sels dans la préparation de médicaments pour des applications thérapeutiques nouvelles.

Elle a pour objet l'utilisation de la metformine pour la préparation d'un médicament destiné au traitement de pathologies du système microvasculaire, notamment les troubles de la vasomotricité spontanée ou de la perméabilité capillaire, ou la néo-vascularisation due à une prolifération anormale des cellules endothéliales.

Il est connu que les troubles de vasomotricité des microvaisseaux, de la microcirculation et de la perméabilité capillaire sont par leur fréquence une cause majeure de pathologies graves et invalidantes.

Peu de substances se sont montrées capables de corriger avec succès des troubles métaboliques et circulatoires liés à l'ischémie et à l'anoxie.

Le composé de l'invention est une base qu'il est possible de salifier, notamment lorsqu'une forme soluble ou rapidement diffusible est désirée, par un acide minéral ou organique pharmaceutiquement acceptable. On citera en particulier les chlorhydrates, sulfates, nitrates, phosphates, sulfites, dithionates, acétates, benzoates, citrates, glycolates, glyoxylates, mercapto-acétates, - hydroxybutyrates, pamoates, aspartates, glutamates, pyrrolidone-carboxylates, méthanesulfonates, naphtalènesulfonates, glucose-1-phosphate, chlorophénoxyacétates.

La metformine : N,N-diméthylimidodicarbonimidicdiamide, est connue pour ses propriétés antidiabétiques.

On vient de découvrir que la metformine, et ses sels d'addition exercent un effet complètement distinct des actions déjà connues pour ces produits. Ils stimulent la vasomotricité microartériolaire spontanée, la microcirculation et corrigent les perméabilités capillaires pathologiques.

Ces effets sont sans aucune relation avec les effets déjà établis de ces produits. Ces nouvelles propriétés confèrent à ces composés des propriétés antiischémiques et antianoxiques. Il s'avère donc que dans les cas d'ischémie ou de lésions des capillaires, comme c'est le cas dans les rétinopathies, les composés selon l'invention sont capables d'assurer une redistribution du flux sanguin, de rétablir une oxygénation normale et d'éviter les phénomènes d'extravasement et d'oedème dus à une hyperperméabilité.

Les composés selon l'invention trouvent de ce fait un nouvel emploi en thérapeutique pour antagoniser ou pour corriger les troubles métaboliques et circulatoires liés à l'ischémie et à l'anoxie des tissus. Ces troubles se rencontrent en particulier dans les rétinopathies, les accidents ischémiques aigus ou chroniques, dans les suites d'infarctus cérébral, dans les artérites, la maladie de Reynaud, les coronarites.

Les médicaments fabriqués en vue de cette nouvelle application thérapeutique contiennent une quantité efficace du principe actif dans le traitement de l'ischémie et de l'anoxie tissulaire. Selon le procédé de l'invention une quantité efficace de Metformine, sous forme libre ou salifiée est incorporée à un excipient ou un véhicule inerte non toxique pharmaceutiquement acceptable.

Les médicaments peuvent être présentés sous diverses formes pharmaceutiques. On peut citer à cet effet les formes destinées à l'administration parentérale telles que les solutés ou les suspensions injectables conditionnées en ampoules, en seringues auto-injectables, en flacons multidoses ou en poches pour perfusions veineuses ; les formes pour l'administration orale comme par exemple les comprimés nus ou enrobés, les comprimés effervescents, les sachets, les gélules, les capsules, les dragées, les poudres ou les granulés, les solutés ou les suspensions buvables, les microgranules, les formes à libération prolongée.

Pour le traitement de l'ischémie, de l'anoxie tissulaire et de l'hyperméabilité capillaire, la posologie usuelle varie de 1 à 4 administrations journalières, et la concentration unitaire varie de 100 à 1000 mg de principe actif par prise.

L'invention a également pour objet une autre nouvelle application relevant du traitement des microangiopathies variées liées au développement de réseaux de néovaisseaux.

On a découvert que la Metformine, et ses sels exercent un autre effet complètement distinct des actions déjà connues pour ces produits. Ils s'opposent au phénomène de néovascularisation d'origines diverses par un mécanisme actuellement inconnu. Cet effet est sans aucune relation avec les effets déjà établis de ces produits. Cette nouvelle propriété confère à ces composés une aptitude à s'opposer ou à limiter la progression des lésions vasculaires en particulier au niveau de la rétine où le développement de réseaux néovasculaires entraîne de graves complications vitréorétiniennes. Des phénomènes de même nature apparaissent également lors d'altérations du cartilage ainsi que dans certains territorires tumoraux.

Les composés de l'invention trouvent de ce fait un nouvel emploi en thérapeutique pour lutter contre l'apparition des néovaisseaux ou pour réduire le développement d'une néovascularisation installée. Ces troubles se rencontrent en particulier dans les retinopathies, les cas de gonarthrose, les polyarthrites et en pathologie tumorale.

Les médicaments destinés au traitement ou à la régression d'une néo-vascularisation, contiennent une quantité thérapeutiquement efficace de principe actif dans le dit traitement.

Selon le procédé de l'invention une quantité efficace de Metformine sous forme libre ou de sel, tel que précédemment défini, est incorporée à un excipient ou un véhicule inerte non toxique pharmaceutiquement acceptable.

La quantité efficace de Metformine selon l'invention est comprise entre 100 et 1000 mg par prise unitaire.

La posologie varie selon la voie d'administration et l'indication thérapeutique. La posologie journalière sera en général de 1 à 4 administrations.

Les compositions pharmaceutiques destinées au traitement de la néo-vascularisation sont celles qui conviennent pour l'administration parentérale sous forme de solutés et suspensions injectables conditionnés en ampoules, flacons pour perfusion veineuse lente, ou pour l'administration par voie buccale (sachets, comprimés, effervescents, gélules, comprimés nus ou enrobés, dragées, ampoules ou solutés buvables, microgranules, formes à libération prolongée).

Les exemples suivants illustrent l'invention sans toutefois la limiter.

Exemple I

| Comprimés effervescents à 0,500 g de Metformine : | |
|---|---|
| . metformine chlorhydrate | 641,5 g |
| . lactose | 105,0 g |
| . phosphate monopotassique | 17,5 g |
| . acide citrique | 16,0 g |
| . bicarbonate de sodium | 8,4 g |
| . stéarate de magnésium | 2,1 g |

pour 1 000 comprimés terminés au poids moyen de 0,790 g.

Exemple II

| Forme injectable à 250 mg de | |
|---|---|
| . metformine chlorhydrate | 321 mg |
| . sorbitol | 400 mg |
| . eau p.p.i | q.s.p.  10 ml |

Example III

```
Microgranules à 250 mg de
        . metformine chlorlydrate.............. 321 mg
        . Amidon de maïs...................... 15 mg
        . Gomme laque......................... 19 mg
        . Polyvidone excipient................ 9,5 mg
        . Polyoxyéthylène glycol 4000......... 10 mg
        . Saccharose.......................... 40 mg
        . Talc............................... 19 mg
        . Gélule taille 1 : 1 gélule......... 433,5 mg
```

Exemple IV

Etude pharmacologique sur les propriétés antiischémiques.

La capacité de favoriser la vasomotricité microvasculaire spontanée, la microcirculation et la réduction de la perméabilité capillaire pathologique que maifeste la Metformine, a été mise en évidence par différents test, en particulier celui de la fenêtre cutanée chez le hamster éveillé (A.Colantuoni, S. Bertuglia, and M. Intaglietta, Microvascular Research 28 143.158 (1984).

La fenêtre est éclairée par transmission, on observe le lit capillaire au microscope trioculaire. L'image est transformée en vidéosignal par une caméra. L'enregistrement du diamètre des microvaisseaux est réalisé en continu.

Le produit à étudier est administré par voie intra-veineuse. On observe une augmentation de la fréquence de contractions et dilatations des artérioles terminales qui passe de une par minute chez les témoins, à 10 par minute chez les animaux traités par la Metformine à la dose de 2 mg/100 g par voie intraveineuse.

Sur le modèle de la joue de hamster (A.Colantuoni, P.G. ; Berardi, G. Oréfice. The journal of Nuclear Medicine and allied Sciences, 23, N°1-2, 49, 54 (1979)), l'étude de la microcirculation, par observation au microscope à transmission, révèle chez l'animal anesthésié au pentobarbital, une disparition, des contractions rythmiques des cellules musculaires lisses précapillaires. Après traitement par la Metformine, par voie intraveineuse, à la dose de 1-2 mg/100 g, on constante la réapparition des contractions et relaxations rythmiques des cellules musculaires lisses précapillaires. Cet effet est observé le long des artérioles terminales. Il rétablit un débit pulsé dans les ramifications des capillaires et assure une redistribution du sang dans les micro-vaisseaux.

Sur le même modèle expérimental, l'adaptation d'un système d'observation microfluorimétrique (A. Colantuoni..(1979), (K. Ley and K.E. Arfors. Microvascular research 31 84-99 (1986)) permet d'étudier le passage de macromolécules fluorescentes de l'intérieur des microvaisseaux du lit capillaire au milieu interstitiel.

Le marqueur utilisé est le "Fluorescein Isothiocyanate Dextran" de poids moléculaire 150.000.

Sur un modèle de perméabilité capillaire accrue, le tableau I montre qu'un traitement par la Metformine, par voie orale à raison de 3 mg/100 g par jour, réduit dans des proportions considérables les pourcentages de cas de perméabilité augmentée observés au bout de 60 et de 90 jours chez les animaux non traités.

EP 0 283 369 B1

## TABLEAU I

| | % DE CAS DE PERMEABILITE AUGMENTEE | |
|---|---|---|
| | A 60 JOURS | A 90 JOURS |
| SANS TRAITEMENT | 60 | 100 |
| METFORMINE V.O. 3 mg/100 g/j. | 0 | 33 |

Dans un modèle d'ischémie périphérique chez le rat, l'injection de microsphères dans l'artère fémorale, provoque l'apparition d'un oedème de la patte ainsi qu'une baisse des capacités motrices de l'animal. Un traitement par la Metformine par voie intra péritonéale à raison de 20 mg/kg réduit l'oedème de 60% et permet la récupération totale de la motricité.

Exemple V

Etude pharmacologique dans le domaine de la néovascularisation.

- la néovascularisation est le résultat d'une prolifération anormale de cellules endothéliales en particulier dans un environnement hypoxique.
  * Cette situation peut être reproduite in vitro en étudiant une telle prolifération sur une culture de cellules endothéliales humaines placées en hypoxie. La Metformine a des concentrationd de l'ordre de $10^{-7}$ à $10^{-12}$ M inhibe fortement cette prolifération. A l'inverse des antimitotiques, la Metformine ne présente pas de cytotoxicité sur ces cultures cellulaires.
  * Cet effet antiangiogénique a été vérifié par 2 modèles d'aniogénèse pathologique in vivo. Tout d'abord dans le modèle classique de néovascularisation de la cornée du lapin (M.A. Gimbrone Jr, R.S. Cotran, S.B. Leafman et A.J. Folkman, J. Natl Cancer Inst. 52). L'administration in situ de Metformine inhibe l'apparition des néovaisseaux.

De plus, la capacité de s'opposer à la formation de néovaisseaux que manifeste la Metformine, a été mise en évidence en particulier sur un modèle expéimental d'étude de la formation de néovaisseaux (C.J. Pournaras, J. Ilic, N.Gilodi. Klin Mbl. Augenheilk. 186 (1985) 471-476). Les expériences ont été partiquées chez le porc miniature. Une occlusion veineuse irréversible a été obtenue par photocoagulation au moyen du laser à Argon. L'angiographie fluorescéinique permet de visualiser 4 semaines après l'occlusion, dans les zones où les vaisseaux de suppléance sont insuffisants en nombre pour assurer la fonction de drainage, le développement d'une néovascularisation. Dans une expérience comparable portant sur 6 porcs miniatures traités par la Metformine, par voie orale à la dose journalière de 100 mg, pendant 4 semaines, on a constaté la suppression complète de l'apparition d'une néovascularisation.

Dans le domaine de la pathologie tumorale, en particulier concernant le processus métastasique, l'effet sur l'endothélium vasculaire laisse à penser que la colonisation extra-vasculaire par des cellules cancéreuses circulantes pourrait être freinée in vitro et in vivo par la Metformine.

5

Effectivement, in vitro la Metformine à la concentration de $10^{-4}$ - $10^{-5}$ M inhibe la pénétration de cellules cancéreuses à travers l'endothélium en culture sur sa matrice extracellulaire.

In vivo, dans un modèle de métastases pulmonaires chez la souris (Cancer chemother. Reports, 1972), la Metformine à 25 et 12,5 mg par voie orale provoque une baisse de 50% du nombre de métastases.

**Revendications**

1. Utilisation de la metformine pour la préparation d'un médicament destiné au traitement de pathologies du système microvasculaire, notamment les troubles de la vasomotricité spontanée ou de la perméabilité capillaire, ou la néovascularisation due à une prolifération anormale de cellules endothéliales.

2. Utilisation de la metformine selon la revendication 1, pour l'obtention d'un médicament destiné au traitement des troubles de la vasomotricité spontanée.

3. Utilisation de la metformine selon la revendication 2, pour l'obtention d'un médicament destiné au traitement des troubles de la vasomotricité spontanée en cas d'hypoxie ou d'anoxie tissulaire, de rétinopathies, d'accidents ischémiques chroniques ou aigus, de séquelles de l'infarctus cérébral, d'artérites, de maladie de Raynaud, de coronarites.

4. Utilisation de la metformine selon la revendication 1, pour l'obtention d'un médicament destiné au traitement des troubles de la perméabilité capillaire.

5. Utilisation de la metformine selon la revendication 4, pour l'obtention d'un médicament destiné au traitement des rétinopathies.

6. Utilisation de la metformine selon la revendication 1, pour l'obtention d'un médicament destiné au traitement de néovascularisation.

7. Utilisation de la metformine selon la revendication 6, pour l'obtention d'un médicament destiné au traitement de la néovascularisation en cas de complications vitréorétinienne résultant du développement de réseaux néovasculaires au niveau de la rétine et des polyarthrites.

8. Utilisation selon l'une des revendications 1 à 7 caractérisée en ce que la metformine est sous forme de sel d'acide minéral ou organique pharmaceutiquement acceptable.

9. Utilisation selon la revendication 8, caractérisée en ce que le sel est choisi parmi les groupes consistant en chlorhydrates, sulfates, nitrates, phosphates, sulfites, dithionates, acétates, benzoates, citrates, glycolates, glyoxylates, mercaptoacétates, $\gamma$-hydroxybutyrates, pamoates, aspartates, glutamates, pyrrolidone-carboxylates, methanesulfonates, naphtalènulfonates, glucose-1 phosphates, et chlorophénoxy-acétates.

10. Utilisation selon l'une des revendications 1 à 9, caractérisée en ce que la metformine est comprise entre 100 et 1000 mg par dose unitaire.

11. Utilisation selon l'une des revendications 1 à 10, caractérisée en ce que le médicament se présente sous forme de solutés et suspensions injectables, comprimés nus, enrobés ou effervescents, sachets, gélules, capsules, dragées, poudres, granulés, solutés et suspensions buvables, microganules et forme à libération prolongée.

**Claims**

1. Use of metformin for the preparation of a medicament intended for the treatment of pathologies of the micro-vascular system, in particular disorders of spontaneous vasomotricity or of capillary permeability, or neovascularization due to an abnormal proliferation of endothelial cells.

2. Use of metformin according to claim 1, for obtaining a medicament intended for the treatment of disorders of spontaneous vasomotricity.

3. Use of metformin according to claim 2, for obtaining a medicament intended for the treatment of disorders of spontaneous vasomotricity in the case of tissular hypoxia or anoxia, retinopathies, chronic or acute ischemic injuries, after-effects of cerebral infarction, arteritis, Raynaud's disease, coronaritis.

4. Use of metformin according to claim 1, for obtaining a medicament intended for the treatment of disorders of capillary permeability.

5. Use of metformin according to claim 4, for obtaining a medicament intended for the treatment of retinopathies.

6. Use of metformin according to claim 1, for obtaining a medicament intended for the treatment of neovascularization.

7. Use of metformin according to claim 6, for obtaining a medicament intended for the treatment of neovascularization in the case of vitreoretinal complications resulting from the development of neovascular networks at the level of the retina and of polyarthritis.

8. Use according to one of claims 1 to 7 characterized in that the metformin is in the form of a pharmaceutically acceptable mineral or organic acid salt.

9. Use according to claim 8, characterized in that the salt is chosen from the groups consisting of hydrochlorides, sulphates, nitrates, phosphates, sulphites, dithionates, acetates, benzoates, citrates, glycolates, glyoxylates, mercaptoacetates, $\gamma$-hydroxybutyrates, pamoates, aspartates, glutamates, pyrrolidone-carboxylates, methanesulphonates, naphthalenesulphonates, 1-glucose phosphates, and chlorophenoxy-acetates.

10. Use according to one of claims 1 to 9, characterized in that the metformin is comprised between 100 and 1000 mg per unit dose.

11. Use according to one of claims 1 to 10, characterized in that the medicament is presented in the form of injectable solutes and suspensions, plain, coated or effervescent tablets, sachets, gelules, capsules, sugar-coated pills, powders, granules, drinkable solutes and suspensions, microgranules and in a slow-release form.

## Patentansprüche

1. Verwendung das Metformins für die Herstellung eines Medikaments, das für die Behandlung von Erkrankungen des Mikro-Gefäßsystems bestimmt ist, insbesondere bei Störungen der spontanen vasomotorischen Kraft, der Kapillarpermeabilität oder der Blutgefäßneubildung aufgrund einer anormalen Wucherung von endothelialen Zellen.

2. Verwendung des Metformins nach Anspruch 1 für die Herstellung eines Medikaments, das für die Behandlung von Störungen der spontanen vasomotorischen Kraft bestimmt ist.

3. Verwendung des Metformins nach Anspruch 2 für die Herstellung eines Medikamente, das für die Behandlung von Störungen der spontanen vasomotorischen Kraft im Fall von Hypoxie oder Anoxie des Gewebes, von Netzhauterkrankungen, chronischen oder akuten Anfällen der Blutleere (Ischämie), Folgen eines Gehirnschlags, von Arterienentzündungen, dor Raynaud'schen-Krankheit, von Coronaritis bestimmt ist.

4. Verwendung des Metformins nach Anspruch 1 für die Herstellung eines Medikamente, das für die Behandlung von Störungen der kapillaren Permeabilität bestimmt ist.

5. Verwendung des Metformins nach Anspruch 4 für die Herstellung eines Medikaments, das für die Behandlung von Netzhauterkrankungen bestimmt ist.

6. Verwendung des Metformins nach Anspruch 1 für die Herstellung eines Medikaments, das für die Behandlung der Blutgefäßneubildung bestimmt ist.

**7.** Verwendung des Metformins nach Anspruch 6 für die Herstellung eines Medikaments, das für die Behandlung der Blutgefäßneubildung im Fall von Netzhautverglasungs-Komplikationen, die aus der Bildung neuer Blutgefäßnetze im Bereich der Retina herrühren und von Polyarthritis bestimmt ist.

**8.** Verwendung nach eine der Ansprüche 1 bis 7, dadurch gekennzeichnet, daß das Metformin in Form eines Salzes einer Mineralsäure oder in organisch-pharmazeutisch verträglicher Form vorliegt.

**9.** Verwendung nach Anspruch 8, dadurch gekennzeichnet, daß das Salz ausgewählt ist aus Gruppen, die aus Chlorhydraten, Sulfaten, Nitraten, Phosphaten, Sulfiten, Dithionaten, Acetaten, Benzoaten, Citraten, Glycolaten, Glyoxylaten, Mercaptoacetaten, $\gamma$-Hydroxybutyraten, Pamoaten, Aspartaten, Glutamaten, Pyrrolidon-Carboxylaten, Methansulfonaten, Naphthalinsulfonaten, Glucose-1-Phosphaten und Chlorphenoxy-Acetaten bestehen.

**10.** Verwendung nach einem dar Ansprüche 1 bis 9, dadurch gekennzeichnet, daß das Metformin von 100 bis 1000 mg pro Einheitsdosis beträgt.

**11.** Verwendung nach einem der Ansprüche 1 bis 10, dadurch gekennzeichnet, daß das Medikament als gelöster Stoff und in Form von injizierbaren Suspensionen, als bloße, beschichtete oder Brausetabletten, Beutelchen, Gelatinekapseln, Kapseln, Dragées, Pulvern, Granulaten, als trinkbarer gelöster Stoff oder als trinkbare Suspension, Mikrogranulat und in einer Form für eine zeitlich gedehnte Freisetzung vorliegt.